# FASCICULE DE BREVET EUROPEEN

(11) **EP 2 683 348 B1**
(45) Date de publication et mention de la délivrance du brevet: **02.01.2019**
(21) Numéro de dépôt: 12705356.9
(22) Date de dépôt: 23.01.2012
(51) Int. Cl.: A61K 8/86, A61Q 19/00, C08L 71/02

(54) **FORMULATION COSMETIQUE CONTENANT UN COPOLYMERE AMPHIPHILE NON HYDROSOLUBLE COMME AGENT EPAISSISSANT**
KOSMETISCHE FORMULIERUNG MIT EINEM WASSERUNLÖSLICHEN AMPHIPHILEN COPOLYMER ALS VERDICKUNGSMITTEL
COSMETIC FORMULATION CONTAINING A WATER-INSOLUBLE AMPHIPHILIC COPOLYMER AS A THICKENER

(30) Priorité: 07.03.2011 FR 1151811
(43) Date de publication de la demande: 15.01.2014
(73) Titulaire: COATEX, 69730 Genay (FR)
(72) Inventeur: SOUZY, Renaud, F-69300 Caluire Et Cuire (FR); SUAU, Jean-Marc, F-69480 Lucenay (FR); KENSICHER, Yves, F-69620 Theize (FR); GUERRET, Olivier, F-46170 Pern (FR)
(74) Mandataire: Balmefrezol, Ludovic Francis Pierre
(86) Numéro de dépôt international: PCT/FR2012/050139
(87) Numéro de publication internationale: WO 2012/120209

(56) Documents cités:
- EP-A1- 1 160 265
- EP-A1- 2 147 940
- WO-A1-03/062288
- FR-A1- 2 898 127
- "ACRYLATES/METHOXY PEG-15 METHACRYLATE COPOLYMER", INTERNET CITATION, 1 janvier 2011 (2011-01-01), page 1, XP007919689, Extrait de l'Internet: URL:http://www.specialchem4cosmetics.com/s ervices/inci/ingredient.aspx? [extrait le 2011-07-11]

## Description

La présente invention concerne des formulations cosmétiques contenant comme agent épaississant des copolymères amphiphiles à structure peigne, à la fois riches en monomère hydrophobe et en monomère polyalkylène glycol. Ils se présentent sous forme de dispersions de particules solides dans l'eau, dont la masse molaire moyenne en poids est de l'ordre du million de grammes par mole. Une fois salifiés, ils acquièrent un caractère hydrosoluble et permettent d'épaissir efficacement une formulation cosmétique les contenant, ladite formulation cosmétique ayant un pH compris entre 5 et 6. Ceci les prédestine à des formulations cosmétiques pour application cutanée, comme notamment les produits de maquillage et de soin du corps.
Nombre de formulations cosmétiques sont constituées majoritairement d'eau et d'une phase non aqueuse, notamment à base d'huiles, de cires, de tensio-actifs, de solvants ou autres excipients. Il s'agit notamment des crèmes, moussants, produits de maquillage, etc... qu'on peut désigner sous l'expression de formulations cosmétiques pour application cutanée, en ce sens qu'elles sont destinées à être appliquées sur la peau. On cherche tout naturellement à épaissir de telles formulations dans une gamme de pH correspondant à celui de la peau, c'est-à-dire à des valeurs comprises entre 5 et 6.
Il existe un certain nombre de solutions techniques à ce problème, qui peuvent être classées en 4 catégories : la mise en oeuvre de polymères de hauts poids moléculaires et sous forme de poudre, la technique dite "retour-acide" qui s'appuie sur des polymères acryliques en émulsion directe de particules polymériques dans l'eau, la mise en oeuvre d'autres polymères toujours sous forme d'émulsions directes, et enfin l'utilisation d'émulsions inverses.
Dans la première catégorie, on peut citer le document EP 1 138 703 A1 qui décrit une composition topique cosmétique, comprenant un polymère de hauts poids moléculaires, à base d'au moins un monomère possédant une fonction acide fort libre copolymérisé avec au moins un monomère estérifié et terminé par un groupe hydrophobe ayant de 8 à 30 atomes de carbone. Le polymère précité est un polymère émulsionnant, sous forme solide ; il peut être dispersé dans l'eau et il permet d'épaissir la composition qui le contient, notamment pour des valeurs de pH voisines de 5.

Toutefois, ces polymères présentent les inconvénients liés à l'emploi d'une poudre : difficultés de transport et de nettoyage, dangerosité du produit en rapport à son caractère pulvérulent, irritant et particulaire. De plus, ces polymères doivent être solubilisés dans le milieu à épaissir, par l'introduction de tensio-actifs. Ces derniers constituent des additifs de formulation supplémentaires qui complexifient la formulation et peuvent interagir avec les tensio-actifs déjà contenus dans ladite formulation, créant parfois des effets indésirables (notamment séparation de phase, formation d'insolubles résiduels).

On connaît aussi la technique dite « retour-acide » (selon l'expression anglophone « back-acid »), telle que décrite dans le document WO 01 / 76 552. Il s'agit d'un procédé consistant à introduire dans un milieu aqueux un tensio actif et un copolymère acrylique alcali gonflable. Ce dernier conduit à un effet épaississant lorsque ses groupements acides carboxyliques sont neutralisés : il y a alors création d'un réseau tridimensionnel qui conduit à une augmentation de la viscosité de la phase aqueuse. Un tel effet peut être déclenché dans une zone de pH voisine de 6, le rôle du tensio-actif étant de maintenir l'effet épaississant, même lorsqu'on fait diminuer le pH.

Au mécanisme ionique précité, peut s'ajouter un mécanisme associatif, reposant sur la présence d'un monomère hydrophobe : c'est ce que décrit le document WO 03 / 62 288, qui vise aussi à épaissir des formulations à pH acide. Il en va de même du document US 4 529 773 A1. Comme pour la méthode de retour-acide, la présence d'un tensio-actif sous forme d'un produit additionnel est donc nécessaire, entraînant les inconvénients déjà évoqués.

On connaît aussi un certain nombre de documents qui décrivent la mise en oeuvre d'autres polymères en émulsion. A ce titre, le document EP 0824914 B1 décrit un polymère contenant un monomère cationique aminé. L'effet épaississant recherché va être obtenu à pH acide via l'ionisation du monomère cationique aminé. Dans le document WO 2004 / 024 779, la cationicité du polymère envisagé est apportée par un monomère vinylique amino substitué. On parvient ici aussi à épaissir un milieu aqueux à pH acide. Cependant, on connaît bien la toxicité des polymères cationiques sur la faune aquatique : or, on les retrouve malheureusement en fin de cycle de vie dans nos fleuves et nos rivières dans lesquels ils sont déversés par l'intermédiaire du réseau d'eau domestique. Enfin, on connaît les émulsions inverses et leurs applications comme agents épaississants dans le domaine de la cosmétique, comme divulgué dans les documents WO 2004 063228 A1 et GB 2422605 A1. Néanmoins, ces structures requièrent la présence de tensio-actifs et de solvants pour assurer leur stabilité, et on fait alors face aux inconvénients mentionnés plus haut.
Aussi, poursuivant ses recherches en vue d'épaissir des compositions aqueuses, notamment à des pH inférieurs à 7, tout en palliant aux inconvénients des méthodes de l'art antérieur, la Demanderesse a mis au point un procédé d'épaississement employant des structures originales : il s'agit de copolymères non hydrosolubles à structure peigne, disposant d'un squelette (méth)acrylique, sur lequel sont greffées des chaînes latérales contenant au moins un monomère hydrophobe du type styrène ou ses dérivés ou ester (méth)acrylique en C1 à C4 et au moins un monomère hydroxy ou méthoxy polyalkylène glycol.
Les teneurs en comonomères sont telles que ces copolymères sont amphiphiles : ils sont à la fois riches en monomère hydrophobe et en monomère polyalkylène glycol. Ils se présentent sous forme de dispersions de particules solides dans l'eau, leur masse molaire moyenne en poids étant comprise entre 1 000 000 et 15 000 000 g/mol. Une fois salifiés, ces polymères augmentent leur solubilité dans l'eau. Ils ont été évoqués pour la première fois dans les Demandes de Brevet Françaises non encore publiées et déposées sous les numéros FR 10 56658 et FR 10 56659. Ces structures sont obtenues par des procédés de polymérisation classiques, mettant en oeuvre des initiateurs connus (voir par exemple les documents EP 1 981 920 A1 et EP 0 819 704 A1).
Ces copolymères permettent d'épaissir efficacement une formulation aqueuse à un pH compris entre 5 et 6, notamment une formulation cosmétique contenant une phase aqueuse et une phase non aqueuse, destinée à une application cutanée.

Aussi, un premier objet de la présente invention consiste en une formulation cosmétique contenant une phase aqueuse et une phase non aqueuse, et caractérisée en ce qu'elle contient au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R - (OE)ₘ - (OP)ₙ - R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant la fonction méthacrylate ou méthacryluréthane,
   - R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R - (OE)ₘ - (OP)ₙ - R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant une fonction polymérisable, préférentiellement la fonction méthacrylate ou méthacryluréthane,
   - R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,

la somme des % a), b), c), d) et e) étant égale à 100 %, et en ce que ladite formulation cosmétique a un pH compris entre 5 et 6.

Cette formulation est aussi caractérisée en ce que ledit copolymère présente une masse molaire moyenne en poids comprise entre 1 000 000 et 15 000 000 g/mol, préférentiellement entre 1 000 000 et 6 000 000 g/mol, telle que déterminée par GPC. On se reportera à la technique de mesure décrite dans le document WO 07 / 069037 A1.

Ledit copolymère est obtenu par des procédés connus de copolymérisation radicalaire conventionnelle en solution, en émulsion directe ou inverse, en suspension ou précipitation dans des solvants appropriés, en présence d'initiateurs et d'agents de transfert connus, ou encore par des procédés de polymérisation radicalaire contrôlée tels que la méthode dénommée Réversible Addition Fragmentation Transfer (RAFT), la méthode dénommée Atom Transfer Radical Polymerization (ATRP), la méthode dénommée Nitroxide Mediated Polymerization (NMP) ou encore la méthode dénommée Cobaloxime Mediated Free Radical Polymerization.

Il est obtenu sous forme acide et éventuellement distillé. Il peut être également partiellement ou totalement neutralisé par un ou plusieurs agents de neutralisation choisis préférentiellement parmi les hydroxydes de sodium et de potassium et leurs mélanges.

Cette formulation est aussi caractérisée en ce qu'elle comprend de :
a) 10 à 99,9 %, de préférence 15 à 99,5 %, de préférence encore 20 à 90 %, et mieux encore 50 à 70 % en poids, par rapport à son poids total, de la phase aqueuse,
b) 0,1 à 90 %, de préférence 0,5 à 85 %, de préférence encore 10 à 80 %, et mieux encore 30 à 50 % par rapport à son poids total, de la phase non aqueuse,
la somme a) + b) étant égale à 100 %.

Cette formulation est aussi caractérisée en ce qu'elle comprend de 0,05 à 10 %, de préférence 0,05 à 5 %, de préférence encore 0,05 à 2 %, et mieux encore 0,1 % à 1 % en poids sec, par rapport à son poids total, dudit copolymère peigne amphiphile.

Cette formulation est aussi caractérisée en ce que sa phase non aqueuse est constituée de corps non miscibles à l'eau, gras liquides à température ambiante (25°C) et/ou gras solides à température ambiante tels que les cires, les corps gras pâteux, les gommes et leurs mélanges. Ces corps gras peuvent être d'origine animale, végétale, minérale ou synthétique. Cette phase aqueuse peut, en outre, contenir des solvants organiques lipophiles.

Cette formulation est aussi caractérisée en ce qu'elle peut en outre contenir, dans une phase particulaire, des pigments et/ou des nacres et/ou des charges habituellement utilisés dans les compositions cosmétiques.

Cette formulation est aussi caractérisée en ce qu'elle peut aussi contenir d'autres matières colorantes choisies parmi les colorants hydrosolubles ou les colorants liposolubles bien connus de l'homme du métier.

Cette formulation est aussi caractérisée en ce qu'elle peut contenir un polymère additionnel qui est un polymère filmogène. Selon la présente invention, on entend par « polymère filmogène », un polymère apte à former à lui seul ou en présence d'un agent auxiliaire de filmification, un film continu et adhérent sur un support, notamment sur les matières kératiniques.

Cette formulation est aussi caractérisée en ce qu'elle peut contenir au moins un agent tensioactif ou émulsionnant.

Enfin, cette formulation est aussi caractérisée en ce qu'elle peut contenir des ingrédients couramment utilisés en cosmétique, tels que les vitamines, les parfums, les agents nacrants, les gélifiants, les oligo-éléments, les adoucissants, les séquestrants, les agents alcalinisants ou acidifiants, les conservateurs, les filtres solaires, les anti-oxydants, les agents anti-chutes des cheveux, les agents anti-pelliculaires, les agents propulseurs, les céramides, les agents moussants, les émollients, les humectants, les agents de texture, les éclaircissants, les agents anti-âge, les agents hydratants, les agents anti-stress et/ou apaisants, les agents dermoprotecteurs, ou leurs mélanges.

Un deuxième objet de la présente invention est l'utilisation, dans une formulation cosmétique telle que précitée, d'au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R-(OE)ₘ-(OP)ₙ-R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant la fonction méthacrylate ou méthacryluréthane,
   - R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R-(OE)ₘ-(OP)ₙ-R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant une fonction polymérisable, préférentiellement la fonction méthacrylate ou méthacryluréthane,
   - R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,
la somme des % a), b), c), d) et e) étant égale à 100 %,
comme agent épaississant de ladite formulation, qui a un pH compris entre 5 et 6.

Cette utilisation est aussi caractérisée en ce que ladite formulation a un pH , préférentiellement compris entre 5,5 et 6.
Un dernier objet de la présente invention est un procédé d'épaississement d'une formulation cosmétique ayant un pH compris entre 5 et 6 par introduction dans celle-ci d'au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R - (OE)ₘ - (OP)ₙ - R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant la fonction méthacrylate ou méthacryluréthane,
   - R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R-(OE)ₘ-(OP)ₙ-R', avec :
   - m et n désignant des entiers inférieurs ou égaux à 150 dont l'un au moins est non nul,
   - OE et OP désignant respectivement l'oxyde d'éthylène et l'oxyde de propylène,
   - R désignant une fonction polymérisable, préférentiellement la fonction méthacrylate ou méthacryluréthane,
   - R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,
la somme des % a), b), c), d) et e) étant égale à 100 %.

Ce procédé est aussi caractérisé en ce que le pH de ladite formulation est préférentiellement compris entre 5,5 et 6.
Les exemples qui suivent permettront de mieux appréhender l'invention, sans pour autant en limiter la portée.

### EXEMPLES

### Exemple 1

Cet exemple porte sur l'épaississement à une valeur de pH de 5,5 d'une composition cosmétique qui est une crème nourrissante pour le corps.

On commence par réaliser une formulation à partir des 2 phases suivantes A et B.
Phase A :
   - DC345 (Dow™ Corning) (5,0 g)
   - DC200/100CS (Dow™ Corning) (4,0 g)
   - Emulium Delta (Gattefosse™) (3,0 g)
   - MOD (Gattefosse™) (3,0 g)
   - Labrafac CC (Gattefosse™) (3,0 g)
   - IPP (Brenntag™) (3,0 g)
   - Phenonip (Clariant™) (0,5 g)
Phase B :
   - eau (qsp 100 g)
   - Epaississant à tester

On réalise chaque phase par mélange de ses différents constituants, sous agitation.
On fait ensuite fondre le mélange A dans un bécher de 150 ml à 70°C sans surchauffe. On chauffe ensuite le mélange B à 70°C. Ce dernier est émulsionné dans le mélange A sous agitation (au moyen d'un dispositif Ultra-Turax) pendant 2 minutes à 6 000 tours par minute. Le pH de la formulation est ajusté à 5,5 grâce à de la soude.
On laisse alors refroidir à température ambiante pour obtenir une formulation d'aspect crémeux.

Le tableau 1 donne la composition (en % en poids de chacun des comonomères) des copolymères selon l'invention qui ont été testés.

**Tableau 1**

| Essai n° | AMA | AA | Meth C₂₂(OE)₂₅ | Meth C₂₀(OE)₃₆ | Meth _{C32}(OE)₂₅ | PEM 3070 | MAMPEG 5000 | AE | p(tBu)Sty |
|---|---|---|---|---|---|---|---|---|---|
| 1 | 8 | 0 | 3,5 | 0 | 0 | 32 | 0 | 56,5 | 0 |
| 2 | 0 | 5,5 | 0 | 0 | 0 | 55 | 0 | 0 | 39,5 |
| 3 | 9 | 0 | 2,5 | 0 | 0 | 33,5 | 0 | 55 | 0 |
| 4 | 9 | 0 | 0 | 0 | 0 | 35 | 0 | 56 | 0 |
| 5 | 10 | 0 | 0 | 0 | 4 | 36 | 0 | 50 | 0 |
| 6 | 9,5 | 0 | 0 | 2,5 | 0 | 32 | 0 | 56 | 0 |
| 7 | 9,5 | 0 | 2,5 | 0 | 0 | 32 | 0 | 56 | 0 |
| 8 | 9,5 | 0 | 2,5 | 0 | 0 | 0 | 32 | 56 | 0 |
| 9 | 9 | 0 | 2 | 0 | 0 | 0 | 45 | 44 | 0 |

avec :
AMA : acide méthacrylique
AA : acide acrylique
Meth C₂₂(OE)₂₅ : monomère de formule R - (OE)ₘ - (OP)ₙ - R', où R désigne la fonction méthacrylate, n = 25, m = 0 et R' est le groupe alkyle linéaire ayant 22 atomes de carbone
Meth C₂₀(OE)₃₆: monomère de formule R - (OE)ₘ - (OP)ₙ - R', où R désigne la fonction méthacrylate, n = 36, m = 0 et R' est le groupe alkyle linéaire ayant 20 atomes de carbone
Meth C₃₂(OE)₂₅ : monomère de formule R - (OE)ₘ - (OP)ₙ - R', où R désigne la fonction méthacrylate, n = 35, m = 0 et R' est le groupe alkyle linéaire ayant 32 atomes de carbone
PEM3070 : monomère de formule R - (OE)ₘ - (OP)ₙ - R', où R désigne la fonction méthacrylate, n = 30, m = 70 et R' est le groupe hydroxy
MAMPEG5000 : monomère de formule R - (OE)ₘ - (OP)ₙ - R', où R désigne la fonction méthacrylate, n = 114, m = 0 et R' est le groupe methoxy
AE : acrylate d'éthyle
p(tBu)Sty : para tertiobutylstyrène

Le PEM3070 est un monomère issu de la technologie comme décrite dans la Demande de Brevet US 6 034 208.
Le MAMPEG5000 est commercialisé notamment par la société Coatex™ sous le nom Norsocryl™ 405.

Chacun des polymères selon les essais n° 1 à 9 est introduit dans la formulation précédente, à raison de 0,7 % en poids sec, par rapport au poids total de ladite formulation.
On mesure alors la viscosité Brookfield à 25°C et à 20 tours par minute de la formulation.
L'essai n° 10 correspond à un témoin ne mettant pas en oeuvre d'épaississant.

Les résultats figurent dans le tableau 2.

**Tableau 2**

| Essai n° | Viscosité (mPa.s) |
|---|---|
| 1 | 5 400 |
| 2 | 8 150 |
| 3 | 5 800 |
| 4 | 5 200 |
| 5 | 6 500 |
| 6 | 5 400 |
| 7 | 6 500 |
| 8 | 5 300 |
| 9 | 5 100 |
| 10 | 3 800 |

Sans épaississant, la formulation selon l'essai n° 10 n'est pas stable.
On constate pour le reste que le polymère selon l'invention permet d'épaissir efficacement, c'est à dire au même niveau qu'un polymère du marché, une formulation cosmétique à un pH égal à 5,5.

## Revendications

1. Formulation cosmétique contenant une phase aqueuse et une phase non aqueuse, et **caractérisée en ce qu'**elle contient au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R - (OE)m - (OP)n - R', avec :
- m et n désignant des entiers inférieurs ou égaux à 150 dont l'un au moins est non nul,
- OE et OP désignant respectivement l'oxyde d'éthylène et l'oxyde de propylène,
- R désignant la fonction méthacrylate ou méthacryluréthane,
- R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R - (OE)m - (OP)n - R', avec
- m et n désignant des entiers inférieurs ou égaux à 150 dont l'un au moins est non nul,
- OE et OP désignant respectivement l'oxyde d'éthylène et l'oxyde de propylène,
- R désignant une fonction polymérisable, préférentiellement la fonction méthacrylate ou méthacryluréthane,
- R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,
la somme des % a), b), c), d) et e) étant égale à 100 %,
et **en ce que** ladite formulation cosmétique a un pH compris entre 5 et 6.

2. Formulation selon la revendication 1, **caractérisée en ce que** ledit copolymère présente une masse molaire moyenne en poids comprise entre 1 000 000 et 15 000 000 g/mol, préférentiellement entre 1 000 000 et 6 000 000 g/mol, telle que déterminée par GPC.

3. Formulation selon une des revendications 1 ou 2, **caractérisée en ce qu'**elle comprend de :
a) 10 à 99,9, de préférence 15 à 99,5, de préférence encore 20 à 90, et mieux encore 50 à 70 % en poids, par rapport à son poids total, de la phase aqueuse,
b) 0,1 à 90, de préférence 0,5 à 85, de préférence encore 10 à 80, et mieux encore 30 à 50 % par rapport à son poids total, de la phase non aqueuse,
la somme a) + b) étant égale à 100 %.

4. Formulation selon une des revendications 1 à 3, **caractérisée en ce qu'**elle comprend de 0,05 à 10, de préférence 0,05 à 5, de préférence encore 0,05 à 2, et mieux encore 0,1 % à 1 % en poids sec, par rapport à son poids total, dudit copolymère peigne amphiphile.

5. Formulation selon une des revendications 1 à 4, **caractérisée en ce que** sa phase non aqueuse est constituée de corps non miscibles à l'eau, gras liquides à température ambiante (25°C) et/ou gras solides à température ambiante tels que les cires, les corps gras pâteux, les gommes et leurs mélanges. Ces corps gras peuvent être d'origine animale, végétale, minérale ou synthétique. Cette phase aqueuse peut, en outre, contenir des solvants organiques lipophiles.

6. Formulation selon une des revendications 1 à 5, **caractérisée en ce qu'**elle peut en outre contenir, dans une phase particulaire, des pigments et/ou des nacres et/ou des charges habituellement utilisés dans les compositions cosmétiques.

7. Formulation selon une des revendications 1 à 6, **caractérisée en ce qu'**elle peut aussi contenir des colorants hydrosolubles ou des colorants liposolubles.

8. Formulation selon une des revendications 1 à 7, **caractérisée en ce qu'**elle peut contenir un polymère filmogène.

9. Formulation selon une des revendications 1 à 8, **caractérisée en ce qu'**elle peut contenir au moins un agent tensioactif ou émulsionnant.

10. Formulation selon une des revendications 1 à 9, **caractérisée en ce qu'**elle peut contenir des vitamines, des parfums, des agents nacrants, des gélifiants, des oligoéléments, des adoucissants, des séquestrants, des agents alcalinisants ou acidifiants, des conservateurs, des filtres solaires, des anti- oxydants, des agents anti-chutes des cheveux, des agents anti-pelliculaires, des agents propulseurs, des céramides, des agents moussants, des émollients, des humectants, des agents de texture, des éclaircissants, des agents anti-âge, des agents hydratants, des agents anti-stress et/ou apaisants, des agents dermoprotecteurs, ou leurs mélanges.

11. Utilisation, dans une formulation cosmétique selon une des revendications 1 à 10, d'au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R - (OE)m - (OP)n - R', avec :
- m et n désignant des entiers inférieurs ou égaux à 150 dont l'un au moins est non nul,
- OE et OP désignant respectivement l'oxyde d'éthylène et l'oxyde de propylène,
- R désignant la fonction méthacrylate ou méthacryluréthane,
- R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R - (OE)m - (OP)n - R', avec
- m et n désignant des entiers inférieurs ou égaux à 150 dont l'un au moins est non nul,
- OE et OP désignant respectivement l'oxyde d'éthylène et l'oxyde de propylène,
- R désignant une fonction polymérisable, préférentiellement la fonction méthacrylate ou méthacryluréthane,
- R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,
la somme des % a), b), c), d) et e) étant égale à 100 %. comme agent épaississant de ladite formulation.

12. Utilisation selon la revendication 11, **caractérisée en ce que** ladite formulation a un pH compris entre 5,5 et 6.

13. Procédé d'épaississement d'une formulation cosmétique ayant un pH compris entre 5 et 6 par introduction dans celle-ci d'au moins un copolymère peigne non hydrosoluble amphiphile constitué de, exprimé en % en poids de chacun des comonomères :
a) de 30 % à 60 % d'au moins un monomère hydroxy et/ou méthoxy polyalkylène glycol, de formule R - (OE)m - (OP)n - R', avec :
- m et n désignant des entiers inférieurs ou égaux à 150 dont l'un au moins est non nul,
- OE et OP désignant respectivement l'oxyde d'éthylène et l'oxyde de propylène,
- R désignant la fonction méthacrylate ou méthacryluréthane,
- R' désignant un groupement hydroxy ou méthoxy,
b) de 20 % à 60 % d'au moins un monomère hydrophobe choisi parmi le styrène et le para-tertio-butyl-styrène, et les esters (méth)acryliques ayant de 1 à 4 atomes de carbone sur le groupement ester,
c) de 0,1 % à 10 % d'au moins un monomère qui est l'acide acrylique et/ou méthacrylique,
d) de 0 à 15 % d'un monomère associatif, de formule R - (OE)m - (OP)n - R', avec
- m et n désignant des entiers inférieurs ou égaux à 150 dont l'un au moins est non nul,
- OE et OP désignant respectivement l'oxyde d'éthylène et l'oxyde de propylène,
- R désignant une fonction polymérisable, préférentiellement la fonction méthacrylate ou méthacryluréthane,
- R' désignant un groupement alkyle ou aryle ou alkylearyle ayant de 8 à 36 atomes de carbone, linéaire ou ramifié,
e) de 0 à 5 % d'un monomère ayant au moins deux insaturations éthyléniques,
la somme des % a), b), c), d) et e) étant égale à 100 %.

14. Procédé selon la revendication 13, **caractérisé en ce que** le pH de ladite formulation est compris entre 5,5 et 6.

## Patentansprüche

1. Kosmetische Formulierung, die eine wässrige Phase und eine nichtwässrige Phase enthält und **dadurch gekennzeichnet ist, dass** sie mindestens ein amphiphiles nicht wasserlösliches Kammcopolymer enthält, das, ausgedrückt in Gew.-% jedes der Comonomere, aufgebaut ist aus:
a) 30 bis 60 % mindestens eines Hydroxy- und/oder Methoxypolyalkylenglykols der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für die Methacrylat- oder Methacrylurethan-Funktion steht,
- R' für eine Hydroxy- oder Methoxygruppe steht,
b) 20 bis 60 % mindestens eines hydrophoben Monomers, das aus Styrol und para-tert-Butylstyrol und Methacrylsäureestern mit 1 bis 4 Kohlenstoffatomen in der Estergruppe ausgewählt ist,
c) 0,1 bis 10 % mindestens eines Monomers, bei dem es sich um Acrylsäure und/oder Methacrylsäure handelt,
d) 0 bis 15 % eines Assoziativmonomers der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für eine polymerisierbare Funktion, vorzugsweise die Methacrylat- oder Methacrylurethan-Funktion, steht,
- R' für eine lineare oder verzweigte Alkyl- oder Aryl- oder Alkylarylgruppe mit 8 bis 36 Kohlenstoffatomen steht,
e) 0 bis 5 % eines Monomers mit mindestens zwei ethylenischen Ungesättigtheiten,
wobei die Summe der %-Werte von a), b), c), d) und e) gleich 100 % ist,
und dass die kosmetische Formulierung einen pH-Wert zwischen 5 und 6 aufweist.

2. Formulierung nach Anspruch 1, **dadurch gekennzeichnet, dass** das Copolymer eine gewichtsmittlere Molmasse zwischen 1.000.000 und 15.000.000 g/mol, vorzugsweise zwischen 1.000.000 und 6.000.000 g/mol, gemäß Bestimmung mittels GPC aufweist.

3. Formulierung nach Anspruch 1 oder 2, **dadurch gekennzeichnet, dass** sie Folgendes umfasst:
a) 10 bis 99,9 Gew.-%, vorzugsweise 15 bis 99,5 Gew.-%, weiter bevorzugt 20 bis 90 Gew.-% und noch besser 50 bis 70 Gew.-%, bezogen auf ihr Gesamtgewicht, der wässrigen Phase,
b) 0,1 bis 90 %, vorzugsweise 0,5 bis 85 %, weiter bevorzugt 10 bis 80 % und noch besser 30 bis 50 %, bezogen auf ihr Gesamtgewicht, der nichtwässrigen Phase,
wobei die Summe von a) + b) gleich 100 % ist.

4. Formulierung nach einem der Ansprüche 1 bis 3, **dadurch gekennzeichnet, dass** sie 0,05 bis 10 Trockengewichts-%, vorzugsweise 0,05 bis 5 Trockengewichts-%, weiter bevorzugt 0,05 bis 2 Trockengewichts-% und noch besser 0,1 bis 1 Trockengewichts-%, bezogen auf ihr Gesamtgewicht, des amphiphilen Kammcopolymers umfasst.

5. Formulierung nach einem der Ansprüche 1 bis 4, **dadurch gekennzeichnet, dass** ihre nichtwässrige Phase aus nicht mit Wasser mischbaren Fettsubstanzen, die bei Umgebungstemperatur (25 °C) flüssig sind, und/oder Fettsubstanzen, die bei Umgebungstemperatur fest sind, wie Wachsen, pastösen Fettsubstanzen, Gummen und Mischungen davon besteht. Diese Fettsubstanzen können tierischen, pflanzlichen, mineralischen oder synthetischen Ursprungs sein. Diese wässrige Phase kann außerdem lipophile organische Lösungsmittel enthalten.

6. Formulierung nach einem der Ansprüche 1 bis 5, **dadurch gekennzeichnet, dass** sie außerdem in einer Partikelphase Pigmente und/oder Perlmutte und/oder Füllstoffe, die in kosmetischen Zusammensetzungen gewöhnlich verwendet werden, enthalten kann.

7. Formulierung nach einem der Ansprüche 1 bis 6, **dadurch gekennzeichnet, dass** sie auch wasserlösliche Farbmittel oder fettlösliche Farbmittel enthalten kann.

8. Formulierung nach einem der Ansprüche 1 bis 7, **dadurch gekennzeichnet, dass** sie ein filmbildendes Polymer enthalten kann.

9. Formulierung nach einem der Ansprüche 1 bis 8, **dadurch gekennzeichnet, dass** sie mindestens ein Tensid oder mindestens einen Emulgator enthalten kann.

10. Formulierung nach einem der Ansprüche 1 bis 9, **dadurch gekennzeichnet, dass** sie Vitamine, Duftstoffe, Perlglanzmittel, Gelbildner, Spurenelemente, weichmachende Mittel, Sequestriermittel, Alkalinisierungs- oder Ansäuerungsmittel, Konservierungsstoffe, Lichtschutzmittel, Antioxidantien, Mittel gegen Haarausfall, Antischuppenmittel, Treibmittel, Ceramide, Schaummittel, Emollientien, Feuchthaltemittel, Texturmittel, Aufhellungsmittel, Mittel gegen Alterung, Hydratisierungsmittel, Mittel gegen Stress und/oder beruhigende Mittel, Hautschutzmittel oder Mischungen davon enthalten kann.

11. Verwendung mindestens eines amphiphilen nicht wasserlöslichen Kammcopolymers, das, ausgedrückt in Gew.-% jedes der Comonomere, aufgebaut ist aus:
a) 30 bis 60 % mindestens eines Hydroxy- und/oder Methoxypolyalkylenglykols der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für die Methacrylat- oder Methacrylurethan-Funktion steht,
- R' für eine Hydroxy- oder Methoxygruppe steht,
b) 20 bis 60 % mindestens eines hydrophoben Monomers, das aus Styrol und para-tert-Butylstyrol und Methacrylsäureestern mit 1 bis 4 Kohlenstoffatomen in der Estergruppe ausgewählt ist,
c) 0,1 bis 10 % mindestens eines Monomers, bei dem es sich um Acrylsäure und/oder Methacrylsäure handelt,
d) 0 bis 15 % eines Assoziativmonomers der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für eine polymerisierbare Funktion, vorzugsweise die Methacrylat- oder Methacrylurethan-Funktion, steht,
- R' für eine lineare oder verzweigte Alkyl- oder Aryl- oder Alkylarylgruppe mit 8 bis 36 Kohlenstoffatomen steht,
e) 0 bis 5 % eines Monomers mit mindestens zwei ethylenischen Ungesättigtheiten,
wobei die Summe der %-Werte von a), b), c), d) und e) gleich 100 % ist,
in einer kosmetischen Formulierung nach einem der Ansprüche 1 bis 10 als Verdickungsmittel für die Formulierung.

12. Verwendung nach Anspruch 11, **dadurch gekennzeichnet, dass** die kosmetische Formulierung einen pH-Wert zwischen 5,5 und 6 aufweist.

13. Verfahren zum Verdicken einer kosmetischen Formulierung mit einem pH-Wert zwischen 5 und 6 durch Eintragen mindestens eines amphiphilen nicht wasserlöslichen Kammcopolymers, das, ausgedrückt in Gew.-% jedes der Comonomere, aufgebaut ist aus:
a) 30 bis 60 % mindestens eines Hydroxy- und/oder Methoxypolyalkylenglykols der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für die Methacrylat- oder Methacrylurethan-Funktion steht,
- R' für eine Hydroxy- oder Methoxygruppe steht,
b) 20 bis 60 % mindestens eines hydrophoben Monomers, das aus Styrol und para-tert-Butylstyrol und Methacrylsäureestern mit 1 bis 4 Kohlenstoffatomen in der Estergruppe ausgewählt ist,
c) 0,1 bis 10 % mindestens eines Monomers, bei dem es sich um Acrylsäure und/oder Methacrylsäure handelt,
d) 0 bis 15 % eines Assoziativmonomers der Formel R - (OE)ₘ - (OP)ₙ - R', wobei:
- m und n für ganze Zahlen kleiner oder gleich 150 stehen, von denen mindestens eine nicht gleich null ist,
- OE und OP für Ethylenoxid bzw. Propylenoxid stehen,
- R für eine polymerisierbare Funktion, vorzugsweise die Methacrylat- oder Methacrylurethan-Funktion, steht,
- R' für eine lineare oder verzweigte Alkyl- oder Aryl- oder Alkylarylgruppe mit 8 bis 36 Kohlenstoffatomen steht,
e) 0 bis 5 % eines Monomers mit mindestens zwei ethylenischen Ungesättigtheiten,
wobei die Summe der %-Werte von a), b), c), d) und e) gleich 100 % ist.

14. Verfahren nach Anspruch 13, **dadurch gekennzeichnet, dass** der pH-Wert der Formulierung zwischen 5,5 und 6 liegt.

## Claims

1. Cosmetic formulation containing an aqueous phase and a non-aqueous phase and **characterized in that** it contains at least one amphiphilic non-water-soluble comb copolymer consisting of, expressed in % by weight of each of the comonomers:
a) from 30% to 60% of at least one hydroxy and/or methoxy polyalkylene glycol monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating the methacrylate or methacrylurethane function,
- R' designating a hydroxy or methoxy group,
b) from 20% to 60% of at least one hydrophobic monomer chosen from styrene and paratertiobutylstyrene, and (meth)acrylic esters with 1 to 4 carbon atoms on the ester group,
c) from 0.1% to 10% of at least one monomer which is acrylic and/or methacrylic acid,
d) from 0 to 15% of an associative monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating a polymerizable function, preferentially the methacrylate or methacrylurethane function,
- R' designating a linear or branched alkyl or aryl or alkylaryl group with 8 to 36 carbon atoms,
e) from 0 to 5% of a monomer comprising at least two ethylenic unsaturations,
the sum of the percentages a), b), c), d) and e) being equal to 100%,
and **in that** said cosmetic formulation has a pH between 5 and 6.

2. Formulation according to claim 1 **characterized in that** the said copolymer presents a mean molar mass by weight of between 1,000,000 and 15,000,000 g/mol, preferentially between 1,000,000 and 6,000,000 g/mol, as determined by GPC.

3. Formulation according to one of claims 1 or 2 **characterized in that** it comprises from:
a) 10 to 99.9%, preferably 15 to 99.5%, even preferably 20 to 90%, and even better 50 to 70% by weight with respect to its total weight, of the aqueous phase,
b) 0.1 to 90%, preferably 0.5 to 85%, even preferably 10 to 80%, and even better 30 to 50% by weight with respect to its total weight, of the non-aqueous phase,
the sum of a) + b) being equal to 100%.

4. Formulation according to one of claims 1 to 3, **characterized in that** it includes 0.05 to 10%, preferably 0.05 to 5%, even preferably 0.05 to 2%, and even better 0.1% to 1% by dry weight, with respect to its total weight of the said amphiphilic comb copolymer.

5. Formulation according to one of claims 1 to 4 **characterized in that** its non-aqueous phase is composed of bodies that are non-miscible in water, liquid fats at room temperature (25°C) and/or solid fats at room temperature such as waxes, pasty fats, gums and their mixtures. These fats can be of animal, vegetable, mineral or synthetic origin. In addition, the aqueous phase may contain lipophilic organic solvents.

6. Formulation according to one of claims 1 to 5 **characterized in that** it can also contain in a particulate phase, pigments and/or nacres and/or fillers commonly used in cosmetic compositions.

7. Formulation according to one of claims 1 to 6, **characterized in that** it may also contain water-soluble dyes or fat-soluble dyes.

8. Formulation according to one of claims 1 or 7 **characterized in that** it can contain a film-forming polymer.

9. Formulation according to one of claims 1 to 8 **characterized in that** it can contain at least one surface active or emulsifying agent.

10. Formulation according to one of claims 1 to 9 **characterized in that** it may contain vitamins, perfumes, nacrating agents, gelling agents, trace elements, softeners, retention aids, alkalinizing or acidifying agents, preservatives, solar filters, antioxidants, anti hair loss agents, anti-dandruff agents, propellant agents, ceramides, foaming agents, emollients, humectants, texture agents, brighteners, anti-aging agents, moisturizing agents, anti-stress and/or soothing agents, dermoprotective agents, or their mixtures.

11. Utilisation in a cosmetic formulation according to one of claims 1 to 10 of at least one amphiphilic non water-soluble comb copolymer made up of, expressed in % by weight of each of the comonomers:
a) from 30% to 60% of at least one hydroxy and/or methoxy polyalkylene glycol monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating the methacrylate or methacrylurethane function,
- R' designating a hydroxy or methoxy group,
b) from 20% to 60% of at least one hydrophobic monomer chosen from styrene and paratertiobutylstyrene, and (meth)acrylic esters comprising 1 to 4 carbon atoms on the ester group,
c) from 0.1% to 10% of at least one monomer which is acrylic and/or methacrylic acid,
d) from 0 to 15% of an associative monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating a polymerizable function, preferentially the methacrylate or methacrylurethane function,
- R' designating a linear or branched alkyl or aryl or alkylaryl group with 8 to 36 carbon atoms,
e) from 0 to 5% of a monomer with at least two ethylenic unsaturations,
the sum of the percentages a), b), c), d) and e) being equal to 100%,
as a thickening agent of the said formulation.

12. Utilisation according to claim 11 **characterized in that** said formulation has a pH between 5.5 and 6.

13. Process for thickening a cosmetic formulation having a pH between 5 and 6 by the introduction into it of at least one amphiphilic non-water soluble comb copolymer consisting of, expressed in % by weight of each of the comonomers:
a) from 30% to 60% of at least one hydroxy and/or methoxy polyalkylene glycol monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating the methacrylate or methacrylurethane function,
- R' designating a hydroxy or methoxy group,
b) from 20% to 60% of at least one hydrophobic monomer chosen from styrene and paratertiobutylstyrene, and (meth)acrylic esters comprising 1 to 4 carbon atoms on the ester group,
c) from 0.1% to 10% of at least one monomer which is acrylic and/or methacrylic acid,
d) from 0 to 15% of an associative monomer of formula R - (EO)ₘ - (PO)ₙ - R', with:
- m and n designating integers that are less than or equal to 150, with at least one of them being a non-zero,
- EO and PO respectively designating ethylene oxide and propylene oxide,
- R designating a polymerizable function, preferentially the methacrylate or methacrylurethane function,
- R' designating a linear or branched alkyl or aryl or alkylaryl group with 8 to 36 carbon atoms,
e) from 0 to 5% of a monomer comprising at least two ethylenic unsaturations,
the sum of the percentages a), b), c), d) and e) being equal to 100%.

14. Process according to claim 13 **characterized in that** said formulation has a pH between 5.5 and 6.
